Europäisches Patentamt
European Patent Office
Office européen des brevets

(19)

(11) Veröffentlichungsnummer: **0 262 334 A2**

(12) # EUROPÄISCHE PATENTANMELDUNG

(21) Anmeldenummer: **87111134.0**

(51) Int. Cl.⁴: **A61K 31/35**

(22) Anmeldetag: **31.07.87**

(30) Priorität: **01.08.86 US 893076**

(43) Veröffentlichungstag der Anmeldung: **06.04.88 Patentblatt 88/14**

(84) Benannte Vertragsstaaten: **BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Welton, Ann Frances**
**37 Maple Drive**
**North Caldwell, N.J. 07006(US)**

(74) Vertreter: **Lederer, Franz, Dr. et al**
**Van der Werth, Lederer & Riederer**
**Patentanwälte Lucile-Grahn-Strasse 22**
**D-8000 München 80(DE)**

(54) **Entzündungshemmende Mittel.**

(57) Die Verbindung 6-Acetyl-7-[5-(4-acetyl-3-hydroxy-2-propylphenoxy)pentyloxy] -3,4-dihydro-2H-1-benzopyran-2-carbonsäure, sowie Enantiomere, Salze und niedere Alkylester davon, sind in topisch verabreichbaren Dosierungsformen nützlich zur Behandlung von Leuktorien-vermittelten dermalen Entzündungen, wie Psoriasis. Diese Verbindungen können zu Crèmen, Salben, Lotionen, Gelen und anderen für die Anwendung auf der Haut geeigneten Präparatetypen formuliert werden.

FIGUR 1

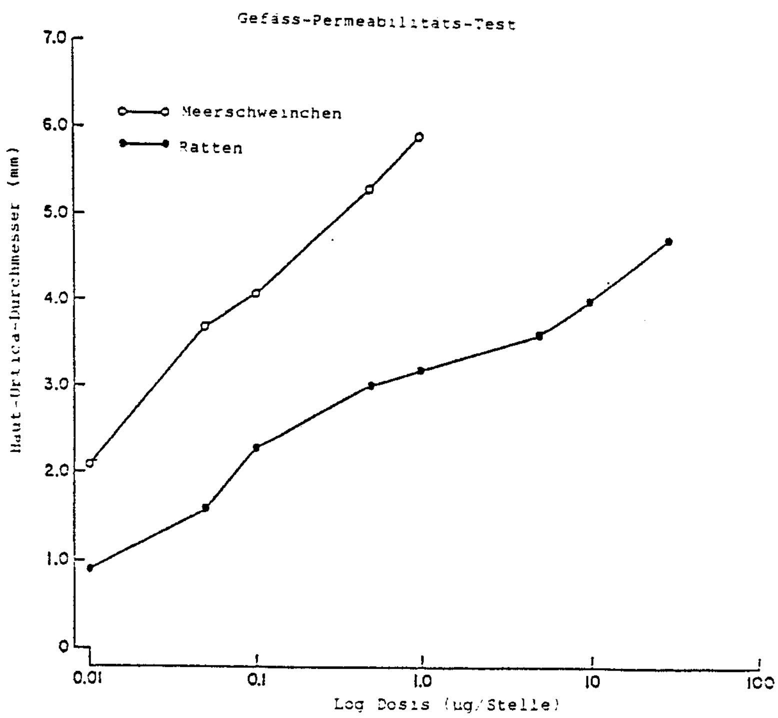


EP 0 262 334 A2

Xerox Copy Centre

## Entzündungshemmende Mittel

Die vorliegende Erfindung betrifft ein einem Aspekt ein Verfahren zur Behandlung von Leukotrien-vermittelten dermalen Entzündungen, das dadurch gekennzeichnet ist, dass man eine wirksame, entzündungshemmende Menge der 6-Acetyl-7-[5-(4-acetyl-3-hydroxy-3-propylphenoxy)pentyloxy] -3,4-dihydro-2H-1-benzopyran-2-carbonsäure oder eines niederen Alkylesters davon, d.h. eine Verbindung der allgemeinen Formel

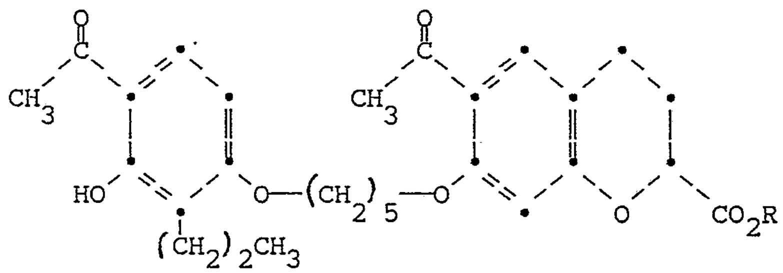

I

worin R Wasserstoff oder niederes Alkyl bedeutet, ein Enantiomer davon oder, sofern R Wasserstoff bedeutet, ein pharmazeutisch annehmbares Salz davon mit einer Base topisch verabreicht.

In einem anderen Aspekt betrifft die vorliegende Erfindung ferner Arzneimittel zur topischen Verabreichung, welche die obigen entzündungshemmenden Verbindungen enthalten und im vorerwähnten Behandlungsverfahren verwendet werden können.

Die erfindungsgemässen Verbindungen und Mittel können zur wirksamen Behandlung von verschiedenen Leukotrien-vermittelten entzündlichen Erkrankungen der Haut, wie Psoriasis, Dermatitis und Ekzemen, verwendet werden.

Bei der Psoriasis handelt es sich um eine chronische Erkrankung der Haut, welche durch eine gesteigerte Proliferation der Epidermis gekennzeichnet ist. Dies führt zu starker Schuppenbildung im befallenen Bereich, Migration von Neutrophilen in die erkrankte Dermis und Epidermis und zu einer Erweiterung und Erhöhung der Permeabilität der Blutgefässe im befallenen Bereich, was sich häufig in abnormalen Rötungen (Erytheme) und wässrigen Schwellungen (Oedemen) manifestiert.

Eine Reihe verschiedener Methoden steht für die Behandlung dieser Krankheit zur Verfügung. Bei topischen Behandlungen werden im allgemeinen Corticosteroide oder Pix-Präparate verwendet, wobei die letzteren manchmal in Verbindung mit Bestrahlungen mit ultraviolettem Licht verwendet werden. Bekannt sind auch systemische Behandlungen mit Corticosteroiden und anderen nicht-topischen antipsoriatischen Mitteln, wie Methotrexat, Hydroxyharnstoff und Etretinat.

Andere Formen entzündlicher Dermatosen, wie Ekzeme und atopische Dermatitis, sind ebenfalls durch die oben erwähnten oder ähnliche Symptome charakterisiert.

Die Verbindungen der Formel I, ihre Enantiomeren und pharmazeutische annehmbaren Salze werden in der publizierten südafrikanischen Patentanmeldung ZA 8404519, 24. Dezember 1984, beschrieben, allerdings für andere Anwendungszwecke, insbesondere zur Verwendung als Antiallergika einschliesslich der Verwendung zur Behandlung von Asthma. Andere Dihydrobenzopyranderivate sind in den europäischen Patentpublikationen Nrs. 139809 (8. Mai 1985) und 150447 (7. Juni 1985) beschrieben, und zwar als Leukotrien-Antagonisten, welche nützlich sind für die Behandlung von Allergien, sowie von entzündlichen Erkrankungen, wie rheumatoide Arthritis. Die vorliegende Erfindung beruht im Gegensatz dazu auf der Erkenntnis, dass die speziellen, hier beschriebenen Verbindungen topisch zur Behandlung von dermalen Entzündungen verwendet werden können, und dass sie in dieser Hinsicht potenter sind als andere Verbindungen aus der gleichen Stoffklasse.

Der Ausdruck "topisch" bedeutet, dass die fraglichen Verbindungen zwecks Ausübung der therapeutischen Wirkung direkt auf die befallene Stelle oder befallenen Stellen der Haut aufgetragen wird. Dies wird zweckmässigerweise dadurch bewerkstelligt, dass man die fraglichen Verbindungen in einen geeigneten pharmazeutischen Träger einarbeitet und das Präparat manuell auf den befallenen Bereich aufträgt. Dies wird weiter unten noch ausführlicher beschrieben werden.

Der Ausdruck "Leukotrien-vermittelt" wird verwendet, um anzudeuten, dass die Substanz Leukotrien an der Verursachung der entzündlichen Hauterkrankung oder an der Manifestation der damit verbundenen Symptome oder an beidem aktiv teilnimmt.

Der Ausdruck "niederes Alkyl" bezeichnet im Rahmen der vorliegenden Erfindung geradkettige oder verzweigte gesättigte Kohlenwasserstoffreste mit 1-7 Kohlenstoffatomen, beispielsweise Methyl, Aethyl, Propyl, Isopropyl, Butyl, t-Butyl, Neopentyl, Pentyl, Hexyl und Heptyl.

Die in der vorliegenden Beschreibung verwendeten Ausdrücke "HAL" und "Halogen" bezeichnen die vier Halogene Brom, Chlor, Fluor und Jod, und zwar vorzugsweise Brom, Chlor und Jod.

Die Verbindungen der Formel I können wie nachfolgend beschrieben gemäss Reaktionsschema I hergestellt werden:

Reaktionsschema I

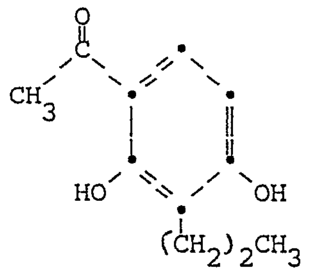

+

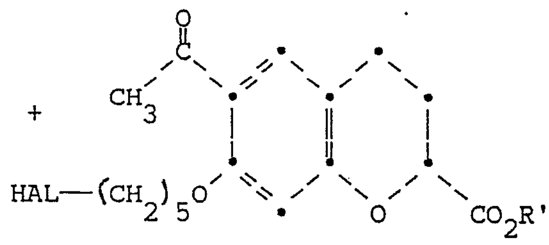

II

III

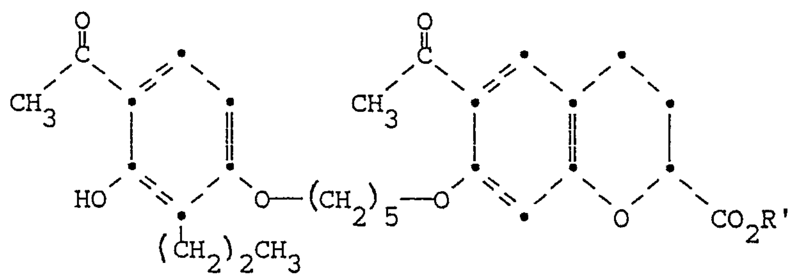

Ia

(optional)

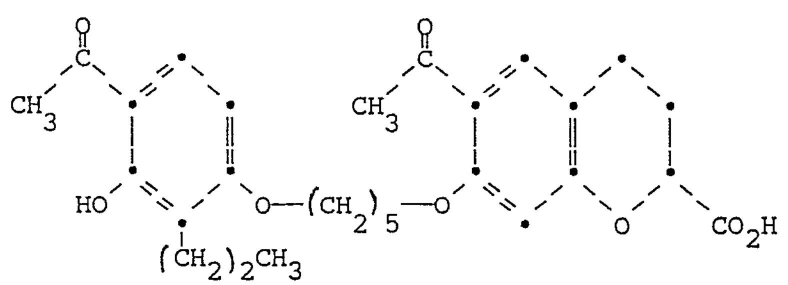

Ib

worin R' niederes Alkyl bedeutet, und HAL die obige Bedeutung besitzt.

Die Umsetzung der Verbindung der Formel II (eine bekannte Verbindung) mit einer Verbindung der Formel III gemäss Reaktionsschema I zu einer Verbindung der Formel Ia wird unter wasserfreien Bedingungen in einem inerten Lösungsmittel, beispielsweise in Aceton, Acetonitril, Methyläthylketon, Diäthylketon, Dimethylformamid oder dergleichen, durchgeführt. Die Reaktion wird bei der Rückflusstemperatur des

Reaktionsgemisches, vorzugsweise bei einer Temperatur im Bereich von etwa 70°C bis etwa 100°C, und in Gegenwart eines säurebindenden Mittels, wie Kaliumcarbonat oder dergleichen, durchgeführt. Als Lösungsmittel verwendet man vorzugsweise ein Gemisch von Aceton und Dimethylformamid. Die erhaltene Verbindung der Formel Ia kann mittels herkömmlicher Methoden, beispielsweise durch Kristallisation, chromatographische Methoden oder dergleichen, isoliert werden.

Die Verbindung der Formel Ia, welche selber erfindungsgemäss verwendet werden kann, kann erwünschtenfalls durch Hydrolyse in eine Verbindung der Formel Ib übergeführt werden. Die Hydrolyse wird mit einem Alkalimetallhydroxid, beispielsweise Lithiumhydroxid, Natriumhydroxid, Kaliumhydroxid oder dergleichen, in einem Gemisch aus Wasser und einem mit Wasser mischbaren Lösungsmittel, beispielsweise Methanol, Aethanol, Tetrahydrofuran oder dergleichen, bei einer Temperatur im Bereich von etwa Raumtemperatur (beispielsweise 20° bis 25°C) bis zur Rückflusstemperatur durchgeführt. Die erhaltene erfindungsgemässe Verbindung der Formel Ib kann mittels herkömmlicher Methoden, beispielsweise durch Extraktion, Kristallisation, chromatogrpahische Methoden der dergleichen, isoliert werden.

Das erfindungsgemässe Verfahren kann auch mit pharmazeutisch annehmbaren Salzen von Verbindungen der Formel I, worin R Wasserstoff bedeutet, durchgeführt werden. Diese Salze können hergestellt werden, indem man eine Säure der Formel I oder ein Enantiomer davon mit einer Base umsetzt, welche ein nicht-toxisches und pharmakologisch und pharmazeutisch annehmbares Kation besitzt. Ganz allgemein umfasst die vorliegende Erfindung Salze mit sämtlichen Basen, welche mit den vorliegenden Carbonsäuren ein Salz bilden und deren pharmakologische Eigenschaften nicht zu nachteiligen physiologischen Reaktionen führen, wenn sie durch einen Warmblüter eingenommen werden. Geeignete Basen sind beispielsweise Alkalimetall-und Erdalkalimetallhydroxide und -carbonate, wie Natriumhydroxid, Kaliumhydroxid, Calciumhydroxid, Kaliumcarbonat und dergleichen, Ammoniak, primäre, sekundäre und tertiäre Amine, wie Monoalkylamine, Dialkylamine, Trialkylamine, stickstoffhaltige heterocyclische Amine, z.B. Piperidin, basische Aminosäuren, wie Lysin, und dergleichen. Die so erhaltenen pharmazeutisch annehmbaren Salze sind funktionelle Aequivalente der entsprechenden 3,4-Dihydro-2H-1-benzopyrancarbonsäure der Formel Ib und dessen Enantiomeren. Dem Fachmann bereitet die Erkennung des Umfanges der therapeutisch verwertbaren, erfindungsgemässen Salze keine Schwierigkeiten, da die Vielfalt durch die vorliegende Erfindung umfassten Salze nur durch die Kriterien beschränkt wird, dass die für die Herstellung dieser Salze verwendeten Basen nicht-toxisch und physiologisch annehmbar sind.

Die für die Herstellung von Verbindungen der Formel I verwendeten Ausgangsstoffe der Formel III sind bekannt oder können, wie nachfolgend beschrieben, gemäss dem Reaktionsschema II hergestellt werden:

Reaktionsschema II

$HAL-(CH_2)_5-HAL$ +

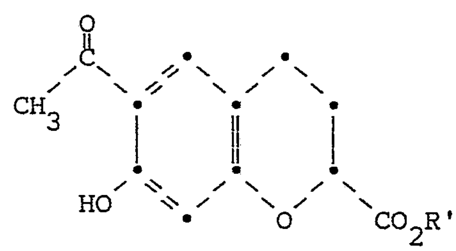

IV V

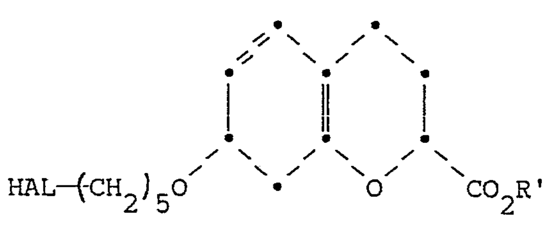

III

worin HAL und R' obige Bedeutung besitzen.

Die Umsetzung einer Verbindung der Formel IV, mit einer Verbindung der Formel V gemäss Reaktionsschema II zu einer Verbindung der Formel III wird in einem inerten organischen Lösungsmittel, wie Dimethylformamid, Aceton, Methyläthylketon, Acetonitril oder dergleichen, vorzugsweise in Acetonitril, durchgeführt. Die Reaktion wird in Gegenwart einer Base, beispielsweise einem Alkalimetallcarbonat, wie Kaliumcarbonat, Natriumcarbonat oder dergleichen, oder einem Alkalimetallhydrid, wie Natriumhydrid oder dergleichen, bei einer Temperatur im Bereich von etwa 20° bis etwa 150°C, vorzugsweise bei Raumtemperatur (z.B. 20° bis 25°C) durchgeführt. Die erhaltene Verbindung der Formel III kann mittels herkömmlicher Methoden, beispielsweise mittels Kristallisation, Extraktion, Chromatographie oder dergleichen, isoliert werden.

Die als Ausgangsstoffe verwendeten verbindungen der Formel V sind bekannt oder können nach an sich bekannten Methoden hergestellt werden.

Die für die Herstellung von Verbindungen der Formel III verwendeten Ausgangsstoffe der Formel V sind bekannt oder können, wie nachfolgend beschrieben, gemäss dem Reaktionsschema III hergestellt werden:

Reaktionsschema III

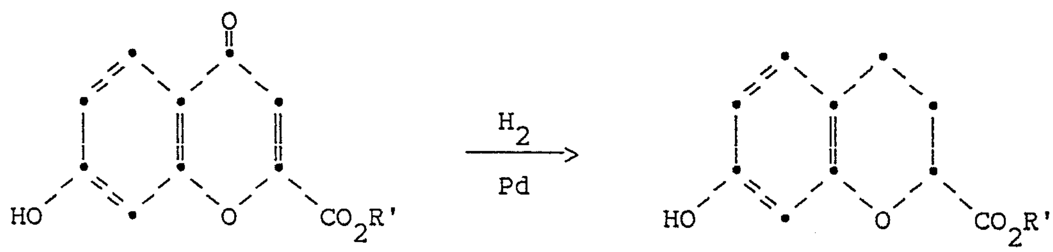


VI Va

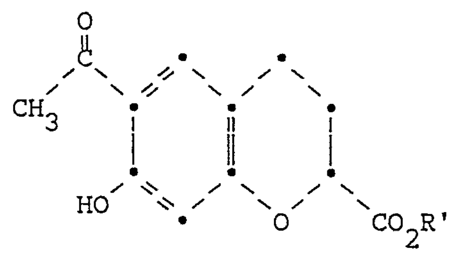

V

worin R' obige Bedeutung besitzt.

Gemäss Reaktionsschema III wird eine Verbindung der Formel VI, welche bekannt ist oder nach an sich bekannten Methoden hergestellt werden kann, zu einer Verbindung der Formel Va hydriert. Im speziellen wird die Reaktion in Gegenwart eines Katalysators, beispielsweise Palladium auf Kohle, in einem Lösungsmittel, wie den niederen Fettsäuren, vorzugsweise in Essigsäure, bei einer Temperatur im Bereich von etwa 20°C bis etwa 100°C, vorzugsweise bei 25°C und bei erhöhtem Druck, vorzugsweise bei 50 psi (344,75 kPa) durchgeführt. Die erhaltene Verbindung der Formel Va kann mittels herkömmlicher Methoden isoliert werden.

Die Verbindung der Formel Va wird anschliessend mittels eines geeigneten Acetylierungsmittels, beispielweise mittels eines Essigsäurehalogenides oder -anhydrides, vorzugsweise Essigsäureanhydrid, in einer geeigneten Base, beispielsweise in einem niederen Alkylamin oder dergleichen, vorzugsweise in Pyridin, bei einer Temperatur im Bereich von etwa 0°C bis etwa 150°C, vorzugsweise bei 25°C, in das entsprechende Acetylderivat der Formel V übergeführt. Die erhaltene Verbindung der Formel V kann mittels herkömmlicher Methoden isoliert werden.

Die Verbindungen der Formel I besitzen ein asymmetrisch substituiertes Kohlenstoffatom und fallen daher normalerweise als racemische Gemische an. Die Spaltung derartiger Racemate in die optische aktiven Isomeren kann nach an sich bekannten Methoden durchgeführt werden. Gewisse racemische Mischungen können als Eutektika ausgefällt und anschliessend aufgetrennt werden. Vorzugsweise wird die Aufspaltung jedoch auf chemischem Wege bewerkstelligt. In diesem Fall werden aus dem racemischen gemisch von Verbindungen der Formel I, worin R Wasserstoff bedeutet, mit einem optisch aktiven Spaltungsmittel die entsprechenden Diastereoisomeren hergestellt. Geeignete Spaltungsmittel sind beispielsweise optisch aktive Basen, wie d-(+)-oder 1-(-)-a-Methylbenzylamin. Die erhaltenen diastereomeren Salze werden durch fraktionierte Kristallisation getrennt und anschliessend in die entsprechenden optischen Isomeren übergeführt. Die vorliegende Erfindung umfasst somit die Verwendung von Racematen von Verbindungen der Formel I als auch ihrer optisch aktiven Isomeren (Enantiomeren).

Weitere Details und spezifischere Methoden für die Herstellung von Verbindungen der Formel I können der weiter oben erwähnten südafrikanischen Patentanmeldung entnommen werden.

Die topischen Arzneimittel, in welche die beschriebenen entzündungshemmenden Verbindungen der Formel I eingearbeitet werden, können vom Typ her in einem breiten Rahmen variieren und umfassen Salben, Crèmes, Lotionen, Gels, usw. Die im Rahmen der vorliegenden Erfindung verwendbaren Lösungen, Salben und Crèmes umfassen Formulierungen mit absorbierbaren, wasserlöslichen Grundlagen oder Grundlagen vom Emulsionstyp, wie Paraffine, Lanolin, Polyäthylenglykole oder dergleichen. Geeignete Lösungen enthalten die Verbindung gelöst in einem pharmazeutisch annehmbaren Lösungsmittel, wie Polyäthylenglykol oder dergleichen.

Geeignete Lotionen sind flüssige Präparate, welche von echten Lösungen bis zu wässrigen oder wässrig/alkoholischen Formulierungen, enthaltend feinverteilte Partikel, variieren können. Diese Lotionen können Suspensions-oder Dispersionsmittel, wie Cellulosederivate, z.B. Methylcellulose, Aethylcellulose, oder dergleichen, enthalten.

Bei den Gelen handelt es sich um halb-feste Präparate, welche durch Gelieren einer Lösung oder Suspension des Wirkstoffes in einer geeigneten wässrigen oder wasserfreien Grundlage erhalten werden, wobei man ein Geliermittel, wie Carboxypolymethylen oder dergleichen, verwendet und anschliessend bis zur geeigneten Konsistenz mit einem Alkalimetallhydroxid, z.B. mit Natriumhydroxid, und einem Amin, z.B. Polyäthylenkokosamin, neutralisiert.

Die erfindungsgemässen topischen Arzneimittel können auch herkömmliche Hilfsstoffe, wie Konservierungsmittel, Stabilisierungsmittel, Netzmittel, Emulgiermittel, Puffer und dergleichen, enthalten. Die benötigten Mengen an Hilfsstoffen richten sich nach den speziellen Erfordernissen und können durch jeden Fachmann leicht ermittelt werden.

Im Sinne einer ergänzenden Beschreibung wird eine Reihe von geeigneten, erfindungsgemässen topischen Arzneimitteln im Rahmen des experimentellen Teiles näher beschrieben.

Repräsentative Dosierungsformen der erfindungsgemässen Arzneimittel enthaltend die beschriebenen Verbindungen in Mengen von etwa 0,1 bis eta 10 Gewichtsprozenten, insbesondere in Mengen von etwa 0,5 bis etwa 5 Gewichtsprozenten, und zwar bezogen auf das Gesamtgewicht (100%) der Dosierungsform. Die in einem gegebenen Fall notwendige minimale oder kleinste wirksame Menge hängt ab von der Stärke der behandelten Krankheit, vom Alter des zu behandelnden Patienten, sowie von der Wirksamkeit, der Konzentration und der Wirkungsdauer der als Wirkstoff eingesetzten Verbindung. In der Regel sollte die entzündungshemmende Menge genügen, um die Symptome der Entzündung abzuschwächen, insbesondere die Rötungen, das Jucken, die Schuppenbildung, die Schwellungen, etc., and den entzündeten Stellen. Das erfindungsgemässe Verfahren wird in der Praxis so durchgeführt, dass man die Salbe, die Crème, die Lotion, das Gel, etc., in einer dünnen Schicht auf die entzündete Fläche aufträgt, leicht einreibt oder aufsprüht, und zwar ein-bis viermal täglich, oder nach Bedarf, je nach der im Warmblüter erwünschten Dosierung.

Die erfindungsgemäss erzielbare, nützliche entzündungshemmende Wirkung kann an Warmblütern unter Verwendung der nachfolgenden Methoden gezeigt werden.

I. Gefäss-Permeabilitäts-Test, in vivo (Ratten und Meerschweinchen

a) Leukotrien ($LTD_4$) alleine

Männliche, 500-600 g schwere Meerschweinchen (Hartley-Stamm) und männliche, 250-300 g schwere Ratten (Sprague-Dawley-Stamm) werden durch intraperitoneale Injektion von 50 mg/kg Körpergewicht Pyrilaminimaleat (ein Antihistaminkum) und 50 mg/kg Mehtylsergid-maleat (ein Serotonin-Antagonist) vorbehandelt. Nach 30 Minuten werden die Versuchstiere mit 1.6 g/kg Urethan anästhetisiert. Unter Verwendung einer Spritze verabreicht man den anästhetisierten Versuchstieren intradermal 0,05 ml einer Kochsalzlösung, welche Leukotrien ($LTD_4$) in Konzentrationen von 0,2 bis 200 µg/ml enthält, und zwar jeweils an vier verschiedenen Stellen. Unmittelbar danach injiziert man 1,0 ml (bei Ratten) bzw. 1,25 ml (bei Meerschweinchen) einer Lösung von 0,5% Evans-Blau in Kochsalzlösung in eine Schwanzvene (bei Ratten) bzw. in eine Ohrvene des rechten Ohres (bei Meerschweinchen). 30 Minuten später werden die Versuchstiere durch Cervikal-Luxation getötet.

Die durch das injizierte Leukotrien bewirkte Zunahme der Gefäss-Permeabilität verursacht eine Migration des Evans-Blau-Farbstoffes in die unmittelbare Umgebung der Stellen, wo das Leukotrien injiziert worden ist. Zur Quantifizierung werden die betroffenen Hautstellen der Versuchstiere operativ entfernt, worauf man die lange und die kurze Achse der verschiedenen blauen Farbflecken mit einer Schublehre mit Nonius ausmisst und aus den erhaltenen Messwerten den durchschnittlichen Durchmesser berechnet. Die Resultate sind in der Figur 1 graphisch dargestellt. Die für die verschiedenen Punkte auf den Kurven berechneten mittleren Durchmesser der Farbflecken basieren auf Messungen an je drei Meerschweinchen bzw. je drei Ratten, wobei für jedes Tier vier Farbflecken ausgemessen worden sind (also insgesamt 12 Auswertungen pro Punkt).

Wie man der Figur 1 entnehmen kann, bewirkt das Leukotrien eine Dosis-abhängige Zunahme der Gefäss-Permeabilität, was sich in einer Migration des Farbstoffes durch die Blutgefässe in dem Bereich der Leukotrien-Injektionspunkte in der Haut der Meerschweinchen und der Ratten widerspiegelt.

b) Leukotrien ($LTD_4$) und Inhibitoren (Bestimmung der Zeitabhängigkeit bei fixer Konzentration)

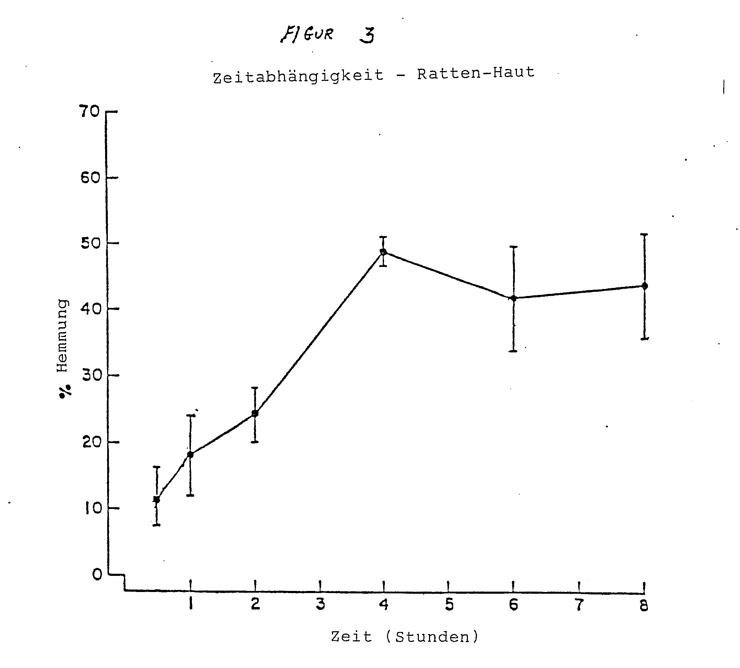

Es wird das Vermögen der racemischen 6-Acetyl-7-[5-(4-acetyl-3-hydroxy-2-propylphenoxy)pentyloxy] -3,4-dihydro-2H-1-benzopyran-2-cargbonsäure bestimmt, die durch intradermale Injektion von Leukotrien bewirkte Erhöhung der Gefäss-Permeabilität zu hemmen. Die zu testende Verbindung wird der gleichen Anzahl Versuchstiere wie oben verabreicht, und zwar indem man einen Tropfen (50 µl) einer Lösung von 10 Gewichtsprozent der zu testenden Verbindung in Dimethylsulfoxid (DMSO) direkt auf die Hautoberfläche appliziert. Dies ergibt eine Menge von 5 mg der zu testenden Verbindung pro Versuchsstelle. Nach Applikation der zu testenden Verbindung wird das Leukotrien ($LTD_4$) nach verschiedenen Zeitintervallen, welche in den Figuren 2 und 3 angegeben sind, intradermal in jede topisch vorbehandelte Stelle der Haut injiziert. Man verwendet dabei jeweils 0,05 ml einer Kochsalzlösung, welche 8 µg/ml Leukotrien enthält, was einer Menge von 0,4 µg Leukotrien pro Versuchsstelle entspricht. Anschliessend wird eine Lösung von Evans-Blau (0,5% in Kochsalzlösung) intravenös in jedes Tiere injiziert, wobei man wie unter Punkt a) beschrieben vorgeht. Nach 30 Minuten werden die Tiere getötet, die behandelten Hautflächen werden entfernt, und das Ausmass der Farbstoff-Migration wird wie oben beschrieben ermittelt. Die wirksamkeit der zu testenden Verbindung, ausgedrückt als Hemmung der durch Leukotrien bewirkten Erhöhung der Permeabilität, wurde gemäss nachfolgender Formel berechnet:

$$\% \text{ Hemmung} = (MUD_t - MUD_c)/MUD_c \times 100$$

wobei $MUD_t$ den bei behandelten Versuchstieren ermittelten mittleren Urtica-Durchmesser bedeutet und $MUD_c$ den bei unbehandelten Kontrolltieren ermittelten mittleren Urtica-Durchmesser bedeutet.

Die durch die zu testende Verbindung in Meerschweinchen und Ratten ermittelte Hemmung in Prozent ist in den Figuren 2 bzw. 3 in Abhängigkeit von der Zeit dargestellt.

Die zu testende Verbindung bewirkt wie gezeigt eine Hemmung der durch Leukotrien bewirkten Erhöhung der Gefäss-Permeabilität, wobei die grösste Aktivität bei Meerschweinchen nach 2-4 Stunden und bei Ratten etwas später erreicht wird.

c) Leukotrien ($LTD_4$) und Inhibitor (Bestimmung der Konzentrationsabängigkeit)

Das Verfahren gemäss Punkt b) wird wiederholt, wobei jedoch die zu testende Verbindung 4 Stunden vor der Leukotrien-Injektion topisch appliziert wird und die Konzentration der zu testenden Verbindung wie in den Tabellen 1 und 2 angegeben variiert wird. Die ermittelten Resultate sind in den Tabelle 1 und 2 zusammengestellt.

Die zu tesende Verbindung hemmt wie gezeigt die durch Leukotrien bewirkte Erhöhung der Gefäss-Permeabilität in einer Dosis-abhängigen Weise.

Tabelle 1

Dosis-abhängige topische Wirkung der zu testenden Verbindung im Gefäss-Permeabilitäts-Test an der Ratte

| Konzentration (% in DMSO | % Hemmung (± SD) |
|---|---|
| 2,5 | 28 ± 4 |
| 5,0 | 50 ± 6 |
| 10,0 | 59 ± 6 |
| 15,0 | NB |
| 25,0 | 67 ± 5 |

NB = nicht bestimmt

Tabelle 2

Dosis-abhängige topische Aktivität der zu testenden Verbindung im Gefäss-Permeabilitäts-Test an Meerschweinchen

| Konzentration (% in DMSO | % Hemmung (± SD) |
|---|---|
| 1,0 | NB |
| 5,0 | 11 ± 3 |
| 10,0 | 33 ± 3 |
| 15,0 | 43 ± 4 |
| 25,0 | 49 ± 4 |

NB = nicht bestimmt

II. Ohr-Oedem-Test an der Maus (in vivo)

In diesem Tiermodellsystem bewirkt die Applikation von Arachidonsäure auf das Ohr die Biosynthese der Metabolite 5-Hydroperoxy-6,8,11,14-eicosatetraencarbonsäure (5-HETE), Leukotrien $B_4$ ($LTB_4$), Leukotrien $C_4$ ($LTC_4$), 12-Hydroperoxy-5,8,10,14-eicosatetraencarbonsäure (12-HETE) und Prostaglandin $E_2$ - ($PGE_2$) am Applikationsort, auf welche ein Zufluss von Neutrophilen an die betroffene Stelle und eine rasche Entwicklung eines Oedems innerhalb von 30-60 Minuten erfolgt (vgl. beispielsweise Young, Wagner und Spires, "Tachyphylaxis in 12-0-Tetradecanoylphorbol Acetate - And Arachidonic Acid - Induced Ear Edema," J. Invest. Dermatol. 80; 48 (1983), und Humes, Opas und Bonney, "Arachidonic Acid Metabolites in Mouse Ear Edema", Advances in Inflammation Research, 11; 57 (1986)). Hemmer dieser Metabolite und ihrer metabolischen Wege hemmen auch die Bildung von Oedemen.

Man verwendet für diesen Versuch männliche, 15-25 g schwere Mäuse (CD-1), welche wie folgt in Gruppen eingeteilt werden: (1) Kontrollgruppe, welche weder mit Arachidonsäure noch mit der zu testenden Verbindung behandelt wird, (2) Arachidonsäure-Gruppe, welche nicht mit der zu testenden Verbindung behandelt wird und (3) behandelte Gruppe, welche zuerst mit der zu testenden Verbindung und anschliessend mit Arachidonsäure behandelt wird. Als Testsubstanz wurde wiederum 6-Acetyl-7-[5-(4-acetyl-3-hydroxy-2-propylphenoxy)pentyloxy] -3,4-dihydro-2H-1-benzopyran-2-carbonsäure verwendet.

Im Falle der Versuchstiere aus der Gruppe (3) wurde die in Aceton gelöste Testsubstanz mit einer 25-Mikroliter-Pipette auf die rückseitige Oberfläche des rechten Ohres der Maus aufgetragen, wobei die Dosis gemäss Tabelle 3 variiert wird. Nach 0,5 Stunden (in einigen Fällen) und 4 Stunden (in anderen Fällen) wird die Arachidonsäure in der gleichen Weise wie oben beschrieben auf die vorbehandelten Ohrflächen topisch appliziert. Es wurde jeweils eine Menge von 0,5 mg Arachidonsäure in 25 $\mu$l Aceton appliziert. Die Arachidonsäure wurde mittels einer 25-Mikroliter-Pipette auf die rückseitige Oberfläche des rechten Ohres topisch appliziert. Nach 1 Stunde werden die Mäuse durch Inhalation von Kohlendioxid getötet. Mittels Stanzbiopsie gewinnt man aus dem behandelten Ohr jeder Maus eine einheitliche Gewebeprobe (Standarddurchmesser 6 mm). Die Gewebeproben werden auf 0,1 mg genau gewogen. Die Hemmung der Bildung von Ohroedemen in Prozent wurde gemäss der nachfolgenden Formel berechnet:

$$\frac{\text{Gewicht Arachidonsäure-Gruppe} - \text{Gewicht behandelte Gruppe}}{\text{Gewicht Arachidonsäure-Gruppe} - \text{Gewicht Kontrollgruppe}} \times 100$$

Die Resultate sind in der Tabelle 3 zusammengestellt. Die Testsubstanz bewirkt in Mengen bis zu 2 mg wie gezeigt erfindungsgemäss eine signifikante Hemmung der Oedembildung, und zwar sowohl wenn sie 0,5 Stunden als auch 4 Stunden vor der Arachidonsäure appliziert wird.

Tabelle 3

Topische Aktivität des Benzopyranderivates als Inhibitor des durch Arachidonsäure bewirkten Ohroedems in Mäusen

| Menge an Testsubstanz in mg[b] | % Hemmung ( ± SD)[a] 0.5 Stunden Vorbehandlung | 4 Stunden Vorbehandlung |
|---|---|---|
| 0.1 | 19 ± 10 | 26 ± 9 |
| 0.2 | 21 ± 12 | 31 ± 12 |
| 0.5 | 42 ± 12 | 28 ± 9 |
| 1.0 | 63 ± 4 | 37 ± 7 |
| 2.0 | 65 ± 1 | 68 ± 7 |

a Für jede Bestimmung wurden 8 Mäuse verwendet.

b Die angegebenen Mengen wurden jeweils in 25 ml Aceton gelöst und in Form einer Lösung appliziert.

## Beispiel 1

### Topische Formulierung (Lösung)

| Bestandteile | Menge, g/100 ml 1g | 2.5g | 5g |
|---|---|---|---|
| racemische 6-Acetyl-7-[5-(4-acetyl-3-hydroxy-2-propylphenoxy)pentyloxy]-3,4-dihydro-2H-1-benzopyran-2-carbonsäure | 1 g | 2.5 g | 5 g |
| Polyäthylenglykol (PEG) 400 s.q. ad | 100 ml | 100 ml | 100 ml |

* PEG 400 kann durch Isopropylmyristat, Neobee-Oel oder Capmul MCM 90 (Stokely - Van Camp Inc., Mischung von $C_8$-$C_{10}$-Mono- und Diglyceriden) ersetzt werden.

Verfahren:

Man löst die racemische 6-Acetyl-7-[5-(4-acetyl-3-hydroxy-2-propylphenoxy)pentyloxy] -3,4-dihydro-2H-1-benzopyran-2-carbonsäure bei Raumtemperatur und in einem geeigneten Behältnis in Polyäthylenglykol.

## Beispiel 2

### Topische Formulierung (Salbe vom nicht-Emulsionstyp)

| Bestandteile | Menge, g/g 0.1g | 0.5g | 1g |
|---|---|---|---|
| racemische 6-Acetyl-7-[5-(4-acetyl-3-hydroxy-2-propylphenoxy)pentyloxy]-3,4-dihydro-2H-1-benzopyran-2-carbonsäure | 0.1g | 0.5g | 1g |
| weisses Paraffin s.q. | 100 g | 100 g | 100g |

Verfahren :

Man mikronisiert die racemische 6-Acetyl-7-[5-(4-acetyl-3-hydroxy-2-propylphenoxy)pentyloxy] -3,4-dihydro-2H-1-benzopyran-2-carbonsäure mittels einer Strahlmühle unter Verwendung von Luft zu einer Partikelgrösse von 1-10 Mikron und verreibt oder mahlt anschliessend mit dem paraffin zu einer Paste.

## Beispiel 3

### Topische Formulierung (Crème)

| Bestandteile | Menge, g/g | | |
|---|---|---|---|
| | 0.1g | 0.5g | 1g |
| racemische 6-Acetyl-7-[5-(4-acetyl-3-hydroxy-2-propylphenoxy)pentyloxy]-3,4-dihydro-2H-1-benzopyran-2-carbonsäure | 0.1g | 0.5g | 1g |
| weisses Paraffin | 30.0g | 30.0g | 30.0g |
| Mineralöl | 20.0g | 20.0g | 20.0g |
| Tensid (Arlacel 83) | 6.0g | 6.0g | 6.0g |
| Tensid (Myrg 52) | 2.0g | 2.0g | 2.0g |
| Polyäthylenglykol 400 | 25.0g | 25.0g | 25.0g |
| Wasser s.q. ad | 100.0g | 100.0g | 100.0g |

Verfahren:

Die verschiedenen Bestandteile werden entsprechend ihren physikalischen und chemischen Eigenschaften in zwei verschiedene Phasen eingearbeitet. Die Phase 1 wird anschliessend unter Rühren oberhalb Raumtemperatur und in einem geeigneten Behältnis in die Phase 2 eingearbeitet, worauf man langsam auf Raumtemperatur abkühlt.

Andere erfindungsgemässe topische Formulierungen in Form von Salben vom Emulsions-Typ, Lotionen, Gelen, etc., sind ebenfalls möglich und können nach an sich bekannten Standardmethoden hergestellt werden.

**Ansprüche**

1. Eine Verbindung der allgemeinen Formel

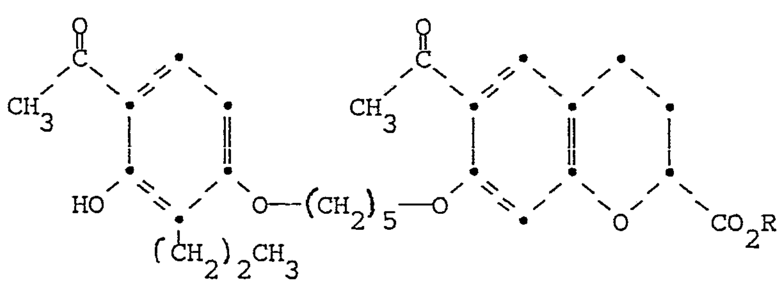

I

worin R Wasserstoff oder niederes Alkyl bedeutet, ein Enantiomeres davon oder, falls R Wasserstoff bedeutet, ein pharmazeutisch annehmbares Salz davon mit einer Base, zur Verwendung als entzündungshemmendes Mittel bei der topischen therapeutischen behandlung von dermalen Entzündungen, welche durch Leukotrien vermittelt werden.

2. Racemische 6-Acetyl-7-[5-(4-acetyl-3-hydroxy-2-propylphenoxy)pentyloxy]-3,4-dihydro-2H-1-benzopyran-2-carbonsäure zur Verwendung als entzündungshemmendes Mittel bei der topischen therapeutischen Behandlung von dermalen Entzündungen, welche durch Leukotrien vermittelt werden.

3. Ein pharmazeutisches, entzündungshemmendes Präparat für die topische Anwendung, enthaltend eine effektiv entzündungshemmende Menge an einer Verbindung der allgemeinen Formel

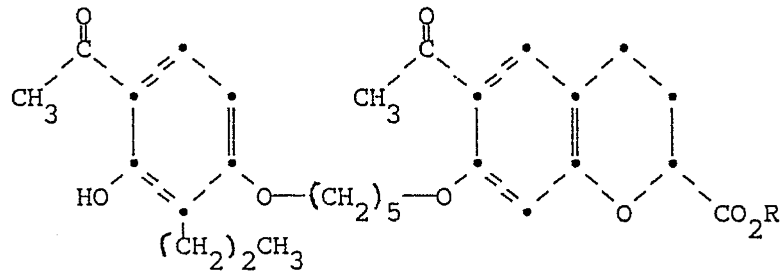

I

worin R Wasserstoff oder niederes Alkyl bedeutet, ein Enantiomeres davon und einen für die topische Anwendung geeigenten, pharmazeutisch annehmbaren Träger.

4. Ein Präparat gemäss Anspruch 3, dadurch gekennzeichnet, dass R Wasserstoff bedeutet.

5. Ein Präparat gemäss Anspruch 3, dadurch gekennzeichnet, dass R niederes Alkyl bedeutet.

6. Ein Präparat gemäss Anspruch 4, dadurch gekennzeichnet, dass man racemische 6-Acetyl-7-[5-(4-acetyl-3-hydroxy-2-propylphenoxy)pentyloxy] -3,4-dihydro-2H-1-benzopyran-2-carbonsäure als Verbindung der Formel I verwendet.

7. Ein Präparat gemäss einem der Ansprüche 3-6, dadurch gekennzeichnet, dass es in der Form einer Lösung, einer Salbe, einer Crème, einer Lotion oder eines Gels vorliegt.

8. Ein Präparat gemäss einem der Ansprüche 3-7, dadurch gekennzeichnet, dass die Konzentration der entzündungshemmenden Verbindung bezogen auf das Gesamtgewicht (100%) des Präparates etwa 0,1 bis etwa 10 Gewichtsprozent beträgt.

9. Ein Präparat gemäss Anspruch 8, dadurch gekennzeichnet, dass die Konzentration der entzündungshemmenden Verbindung bezogen auf das Gesamtgewicht (100%) des Präparates etwa 0,5 bis etwa 5 Gewichtsprozent beträgt.

10. Verwendung einer Verbindung der in Anspruch 1 defininierten Formel I oder eines Enantiomeren davon oder, falls R Wasserstoff bedeutet, eines pharmazeutisch annehmbaren Salzes davon mit einer Base bei der topischen Behandlung von dermalen Entzündungen, welche durch Leukotrien vermittelt werden.

11. Verwendung einer Verbindung der in Anspruch 1 definierten Formel I oder eines Enantiomeren davon oder, falls R Wasserstoff bedeutet, eines pharmazeutisch annehmbaren Salzes davon mit einer Base, zur Herstellung von topischen Präparaten gegen dermale Entzündungen, welche durch Leukotrien vermittelt werden.

12. Verwendung von racemischer 6-Acetyl-7-[5-(4-acetyl-3-hydroxy-2-propylphenoxy)pentyloxy] -3,4-dihydro-2H-1-benzopyran-2-carbonsäure gemäss Anspruch 10 oder 11.

FIGUR 1

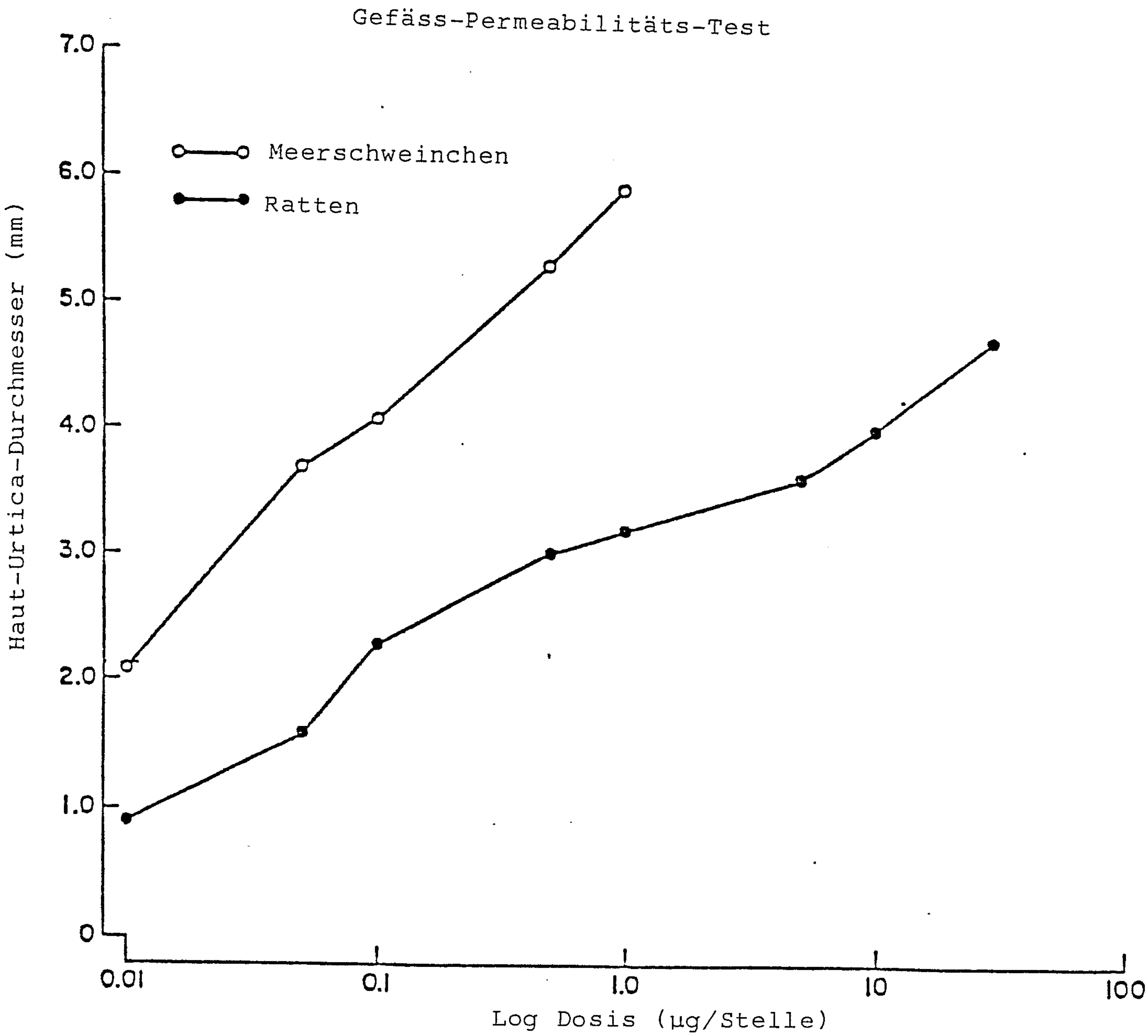


RAN 4050/28

FIGUR 2

Zeitabhängigkeit-Meerschweinchen-Haut

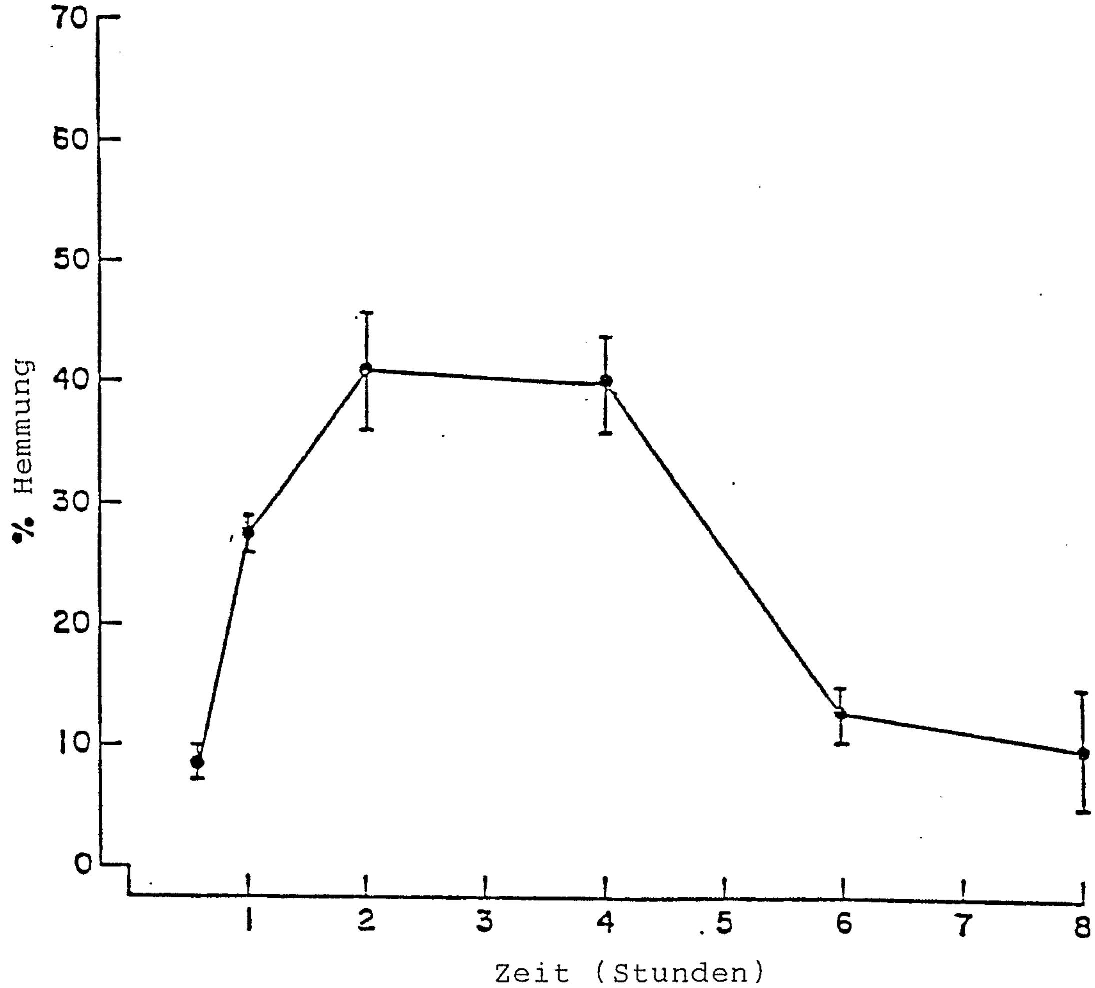


RAN 4050/28

FIGUR 3

Zeitabhängigkeit - Ratten-Haut